(19)

Europäisches Patentamt

European Patent Office

Office européen des brevets

(11) **EP 2 293 245 A1**

(12) **EUROPEAN PATENT APPLICATION**

(43) Date of publication:
**09.03.2011 Bulletin 2011/10**

(51) Int Cl.:
**G06T 7/00** (2006.01)

(21) Application number: **10170226.4**

(22) Date of filing: **21.07.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **31.07.2009 KR 20090070981**

(71) Applicants:
• **Medison Co., Ltd.**
  **Kangwon-do 250-870 (KR)**
• **Korea Advanced Institute of Science and Technology**
  **305-338 Daejeon (KR)**

(72) Inventors:
• **Hyun, Dong Gyu**
  **Seoul 135-851 (KR)**
• **Ra, Jong Beom**
  **Daejon 305-762 (KR)**
• **Lee, Duhgoon**
  **Daejon 302-170 (KR)**
• **Nam, Woo Hyun**
  **Busan 616-806 (KR)**

(74) Representative: **Schmid, Wolfgang**
  **Lorenz & Kollegen**
  **Patentanwälte Partnerschaftsgesellschaft**
  **Alte Ulmer Strasse 2**
  **89522 Heidenheim (DE)**

(54) **Providing a 2-dimensional CT image corresponding to a 2-dimensional ultrasound image**

(57)    There is disclosed a system and method for providing a 2-dimensional CT image corresponding to a 2-dimensional ultrasound image by performing an image registration between a 3-dimensional ultrasound image and a 3-dimensional CT image. The system comprises: a CT image forming unit configured to form a plurality of 3-dimensional CT images for an object of interest inside a target object; an ultrasound image forming unit configured to form at least one 3-dimensional ultrasound image for the object of interest; a processor configured to perform image registration between the 3-dimensional CT images and the at least one 3-dimensional ultrasound image to obtain a first transform function; and a user input unit configured to receive input information from a user, wherein the ultrasound image forming unit is further configured to form a 2-dimensional ultrasound image from the at least one 3-dimensional ultrasound image based on the input information, and wherein the processor is further configured to obtain a plurality of 2-dimensional CT images from the 3-dimensional CT images based on the input information and the first transform function and to detect similarities between the 2-dimensional ultrasound image and the 2-dimensional CT images to select one of the 2-dimensional CT images corresponding to the 2-dimensional ultrasound image.

FIG. 6

```
        START
          │
  FORM 3-DIMENSIONAL CT IMAGES   ── S102
          │
       INTERPOLATION             ── S104
          │
  FIX THE ULTRASOUND PROBE       ── S106
          │
  FORM 3-DIMENSIONAL
  ULTRASOUND IMAGES              ── S108
          │
  EXTRACT ANATOMICAL FEATURES    ── S110
          │
  IMAGE REGISTRATION             ── S112
          │
  RECEIVE INPUT INFORMATION      ── S114
          │
  FORM A 2-DIMENSIONAL
  ULTRASOUND IMAGE               ── S116
          │
       TRANSFORM                 ── S118
          │
  DETECT SIMILARITIES            ── S120
          │
  SELECT A 2-DIMENSIONAL CT IMAGE ── S122
          │
        DISPLAY                  ── S124
          │
         END
```

**Description**

CROSS-REFERENCE TO RELATED APPLICATIONS

The present application claims priority from Korean Patent Application No. 10-2009-70981 filed on July 31, 2009, the entire subject matter of which is incorporated herein by reference.

TECHNICAL FIELD

The present disclosure relates to ultrasound image processing, and more particularly to an image registration-based system and method for providing a 2-dimensional computerized tomography (CT) image corresponding to a 2-dimensional ultrasound image.

BACKGROUND

Due to its non-invasive and non-destructive nature, an ultrasound system has been extensively used in the medical field to acquire internal information of a target object. The ultrasound system is highly useful in the medical field since it can provide doctors with a high resolution image of internal tissues of the target object without the need of surgical treatment.

However, since the signal-to-noise ratio (SNR) of an ultrasound image is low, the method of performing image registration between a CT image and an ultrasound image to provide a CT image and an ultrasound image has been used.

Conventionally, a sensor was used to perform image registration between a CT image and an ultrasound image. Accordingly, the sensor became essential to the system. In addition, there is a problem in that errors can occur when internal organs are transformed due to a movement of the target object such as respiration, etc. Conventionally, when the ultrasound probe is moved to another location to acquire a 2-dimensional ultrasound image, there is a problem in that the sensor is essential to identify whether the 2-dimensional ultrasound image is an ultrasound image within the 3-dimensional ultrasound image or to detect the 2-dimensional CT image corresponding to the 2-dimensional ultrasound image in the 3-dimensional CT image, which has been image registered onto the 3-dimensional ultrasound image.

SUMMARY

The present invention provides a system and method for performing image registration between a 3-dimensional ultrasound image and a 3-dimensional CT image and detecting a 2-dimensional CT image corresponding to a 2-dimensional ultrasound image on the image-registered 3-dimensinal CT image, thereby providing the 2-dimensional CT image without using a sensor.

According to an aspect of the present invention, the image providing system comprises: a CT image forming unit configured to form a plurality of 3-dimensional CT images for an object of interest inside a target object; an ultrasound image forming unit configured to form at least one 3-dimensional ultrasound image for the object of interest; a processor configured to perform image registration between the plurality of 3-dimensional CT images and the at least one 3-dimensional ultrasound image to obtain a first transform function; and a user input unit configured to receive input information from a user, wherein the ultrasound image forming unit is further configured to form a 2-dimensional ultrasound image from the at least one 3-dimensional ultrasound image based on the input information, and wherein the processor is further configured to obtain a plurality of 2-dimensional CT images from the plurality of 3-dimensional CT images based on the input information and the first transform function and to detect similarities between the 2-dimensional ultrasound image and the plurality of 2-dimensional CT images to select one of the 2-dimensional CT images corresponding to the 2-dimensional ultrasound image.

According to another aspect of the present invention, the image providing method comprises: forming a plurality of 3-dimensional CT images for an object of interest inside a target object; forming at least one 3-dimensional ultrasound image for the object of interest; performing image registration between the plurality of 3-dimensional CT images and the at least one 3-dimensional ultrasound image to obtain a first transform function; receiving input information from a user; forming a 2-dimensional ultrasound image from the at least one 3-dimensional ultrasound image based on the input information; obtaining a plurality of 2-dimensional CT images from the plurality of 3-dimensional CT images based on the input information and the first transform function; and detecting similarities between the 2-dimensional ultrasound image and the plurality of 2-dimensional CT images to select one of the 2-dimensional CT images corresponding to the 2-dimensional ultrasound image.

The present invention may provide a 2-dimensional CT image corresponding to a 2-dimensional ultrasound image within a 3-dimensional ultrasound image on a 3-dimensional CT image registered onto the 3-dimensional ultrasound image without using a sensor.

BRIEF DESCRIPTION OF THE DRAWINGS

Figure 1 is a block diagram showing an arrangement of an image providing system according to an embodiment of the present invention.

Figure 2 is a block diagram showing an arrangement of the ultrasound image forming unit according to an embodiment of the present invention.

Figure 3 is a schematic diagram showing an ultrasound probe holder and an ultrasound probe fixed at the ultrasound probe holder according to an embodiment of the present invention.

Figure 4 is a block diagram showing an arrangement of the processor according to an embodiment of the present invention.

Figure 5 is an exemplary diagram showing Hessian matrix eigen values according to directions.

Figure 6 is a flow chart showing the process of providing a 2-dimensional CT image corresponding to a 2-dimensional ultrasound image by performing image registration between a 3-dimensional ultrasound image and a 3-dimensional CT image according to an embodiment of the present invention.

DETAILED DESCRIPTION

Embodiments of the present invention are described below with reference to the accompanying drawings. The term "object of interest" used in this embodiment may comprise a liver inside a target object.

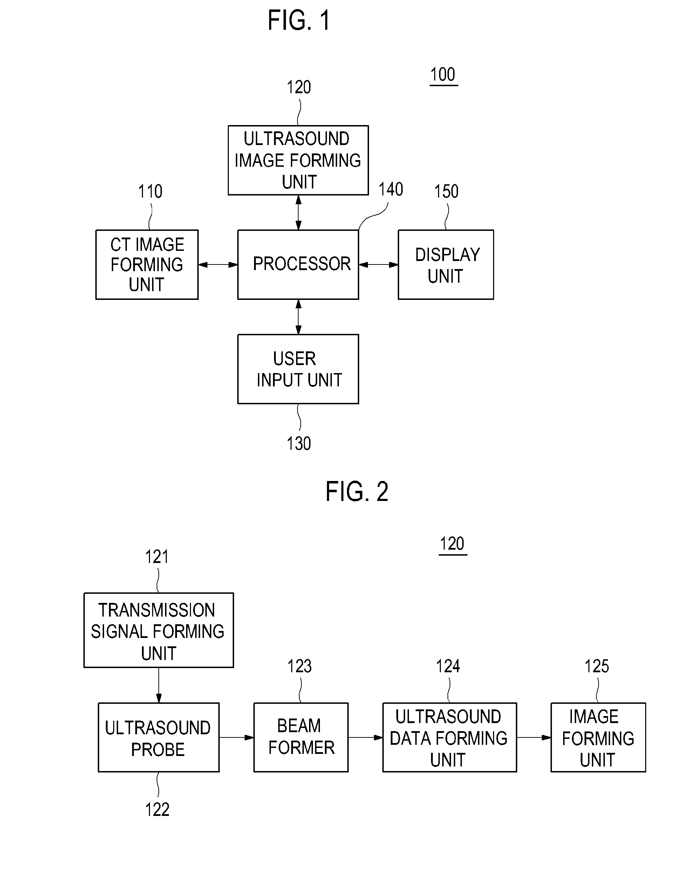

Figure 1 is a block diagram showing an arrangement of an image providing system 100 according to an embodiment of the present invention. The image providing system 100 comprises a computerized tomography (CT) image forming unit 110, an ultrasound image forming unit 120, a user input unit 130, a processor 140 and a display unit 150.

The CT image forming unit 110 forms a 3-dimensional CT image for an object of interest inside a target object, which is composed of a plurality of 2-dimensional CT images. In this embodiment, the CT image forming unit 110 may be configured to consecutively form 3-dimensional CT images $I_{CT}$ $(t_i)$ $(1 \leq i \leq K)$ at a predetermined interval during a respiratory cycle from inspiration to expiration.

The ultrasound image forming unit 120 forms a 3-dimensional ultrasound image for the object of interest inside the target object. In this embodiment, the ultrasound image forming unit 120 forms 3-dimensional ultrasound images $I_{US}$ $(t_j)$ $(1 \leq j \leq 2)$ at maximum inspiration and maximum expiration. Further, the ultrasound image forming unit 120 forms a 2-dimensional ultrasound image of the object of interest inside the target object.

Although it is explained to form the 3-dimensional ultrasound images $I_{US}$ $(t_j)$ $(1 \leq j \leq 2)$ at maximum inspiration and maximum expiration in the foregoing embodiment, the 3-dimensional image can be formed at either maximum inspiration or maximum expiration in other embodiments. In the following description, it is explained that the ultrasound image forming unit 120 forms the 3-dimensional ultrasound images $I_{US}$ $(t_j)$ $(1 \leq j \leq 2)$ at maximum inspiration and maximum expiration for the brevity of the description.

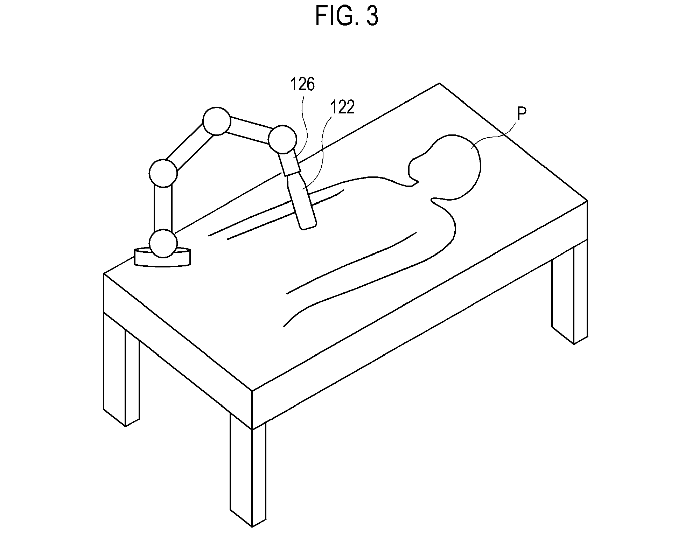

Figure 2 is a block diagram showing an arrangement of the ultrasound image forming unit 120 according to an embodiment of the present invention. The ultrasound image forming unit 120 comprises a transmission signal forming unit 121, an ultrasound probe 122, a beam former 123, an ultrasound data forming unit 124 and an image forming unit 125. The ultrasound image forming unit 120 may comprise an ultrasound probe holder 126 for fixing the ultrasound probe 122 in a specific location of the target object (P) as described in Figure 3.

The transmission signal forming unit 121 forms a first transmission signal to acquire each of the plurality of frames. In this embodiment, the first transmission signal comprises at least one among the transmission signal to acquire each of the plurality of frames at maximum inspiration and the transmission signal to acquire each of the plurality of frames at maximum expiration. Also, the transmission signal forming unit 121 forms a second transmission signal to acquire a frame. The frame may comprise a brightness mode (B-mode) image.

The ultrasound probe 122 comprises multiple transducer elements (not shown). The ultrasound probe 122 may comprise a 3-dimensional probe. However, it should be noted herein that the ultrasound probe 122 may not be limited thereto. The ultrasound probe 122 converts the first transmission signal provided from the transmission signal forming unit 121 into an ultrasound signal, transmits the ultrasound signal to the target object and receives an ultrasound echo signal reflected by the target object to thereby form a first reception signal. In addition, the ultrasound probe 122 moves the transducer elements to the position set by the user. The ultrasound probe 122 then converts the second transmission signal provided from the transmission signal forming unit 121 into an ultrasound signal, transmits the ultrasound signal to the target object and receives an ultrasound echo signal reflected by the target object, thereby forming a second reception signal.

If the first reception signal is provided from the ultrasound probe 122, then the beam former 123 analog/digital converts the first reception signal to form a first digital signal. The beam former 123 forms a first receive-focused signal by receive-focusing the first digital signal considering the focal points and the locations of the transducer elements. If the second reception signal is provided from the ultrasound probe 122, then the beam former 123 analog/digital converts the second reception signal to form a second digital signal. The beam former 123 forms a second receive-focused signal by receive-focusing the second digital signal considering the focal point and the location of the transducer elements.

The ultrasound data forming unit 124 forms first ultrasound data using the first receive-focused signal when the first receive-focused signal is provided from the beam former 123. The ultrasound data forming unit 124 forms second ultrasound data using the second receive-focused signal when the second receive-focused signal is provided from the beam former 123. In addition, the ultrasound data forming unit 124 may perform signal processing, which is required to form ultrasound data (e.g., gain control, filtering, etc.) on the first or second receive-focused signal.

The image forming unit 125 forms a 3-dimensional ultrasound image using the first ultrasound data when the first ultrasound data is provided from the ultrasound data forming unit 124. In this embodiment, the 3-dimensional ultrasound image comprises at least one of the 3-dimensional ultrasound image at maximum inspiration ($I_{US}(t_1)$) and the 3-dimensional ultrasound image at maximum expiration ($I_{US}(t_2)$). The image forming unit 125 forms the 2-dimensional ultrasound image using the second ultrasound data when the second ultrasound data is provided from the ultrasound data forming unit 124.

Referring back to Figure 1, the user input unit 130 receives input information from the user. In this embodiment, the input information comprises reference plane setting information that sets a reference plane in the 3-dimensional ultrasound image across which the 2-dimensional ultrasound image will be obtained, diaphragm area setting information which sets diaphragm areas on the 3-dimensional CT images $I_{CT}(t_i)$ ($1 \leq i \leq K$) and blood vessel area setting information which sets blood vessels area on the 3-dimensional CT images $I_{CT}(t_i)$ ($1 \leq i \leq K$). As an example, the reference plane setting information may comprise reference plane setting information that sets one rotation angle within the rotation angle range in which the transducer element of the ultrasound probe 122 (i.e., 3-dimensional probe) can swing (i.e., -35° to 35°). Thus, the ultrasound image forming unit 120 can form the 2-dimensional ultrasound image corresponding to the reference plane setting information. The user input unit 130 may be implemented with control panel which comprises dial button and the like, mouse, keyboard, etc.

The processor 140 performs image registration between 3-dimensional CT image and 3-dimensional ultrasound image to obtain a transform function between the 3-dimensional CT image and the 3-dimensional ultrasound image (i.e., location of ultrasound probe 122 $T_{probe}$). Hereinafter, it is explained that the 3-dimensional ultrasound images $I_{US}(t_j)$ ($1 \leq j \leq 2$) comprise the 3-dimensional ultrasound image at maximum inspiration ($I_{US}(t_1)$) and the 3-dimensional ultrasound image at maximum expiration ($I_{US}(t_2)$). However, it should be noted that the present invention is not limited thereto. In addition, the processor 140 detects a 2-dimensional CT image corresponding to the 2-dimensional ultrasound image using the transform function.

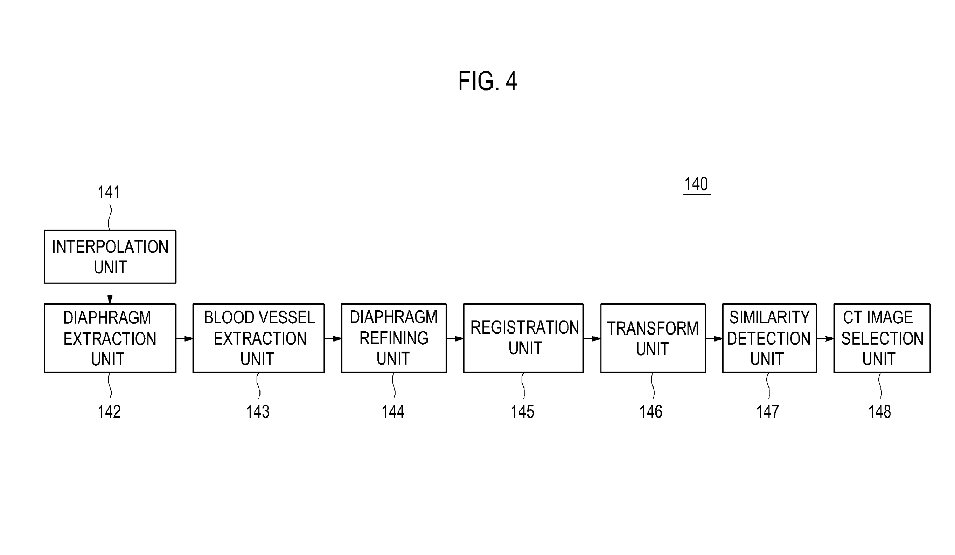

Figure 4 is a block diagram showing an arrangement of the processor 140 according to an embodiment of the present invention. The processor 140 comprises an interpolation unit 141, a diaphragm extraction unit 142, a blood vessel extraction unit 143, a diaphragm refining unit 144, a registration unit 145, a transform unit 146, a similarity detection unit 147 and a CT image selection unit 148.

The interpolation unit 141 interpolates the 3-dimensional CT image $I_{CT}(t_i)$ and the 3-dimensional CT image $I_{CT}(t_{i+1})$ provided from the CT image forming unit 110 to form at least one 3-dimensional CT image between the 3-dimensional CT image $I_{CT}(t_i)$ and the 3-dimensional CT image $I_{CT}(t_{i+1})$. As an example, the interpolation unit 141 performs interpolation between the 3-dimensional CT images $I_{CT}(t_i)$ ($1 \leq i \leq K$) provided from the CT image forming unit 110 to acquire N 3-dimensional CT images $I_{CT}(t_i)$ ($1 \leq i \leq N$).

The diaphragm extraction unit 142 extracts a diaphragm from each of the 3-dimensional CT images $I_{CT}(t_i)$ ($1 \leq i \leq N$) provided from the interpolation unit 141. In addition, the diaphragm extraction unit 142 extracts a diaphragm from the 3-dimensional ultrasound images $I_{US}(t_j)$ ($1 \leq j \leq 2$) provided from the ultrasound image forming unit 120.

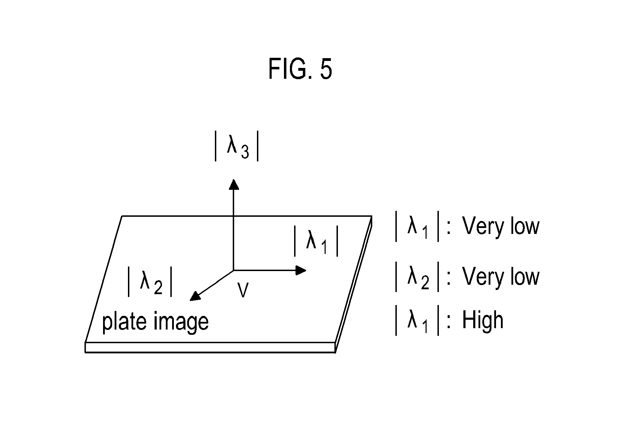

In one embodiment, the diaphragm extraction unit 142 perform a flatness test on the 3-dimensional CT images $I_{CT}(t_i)$ ($1 \leq i \leq N$) and the 3-dimensional ultrasound images $I_{US}(t_j)$ ($1 \leq j \leq 2$) based on a Hessian matrix to extract the diaphragm. That is, the diaphragm extraction unit 142 extracts an area in which the change in the voxel intensity perpendicular to the surface is larger than the change in the voxel intensity parallel to the surface as the diaphragm, considering that the diaphragm is a curved surface on the 3-dimensional CT image and the 3-dimensional ultrasound image. Figure 5 shows Hessian matrix eigen values $\lambda_1$, $\lambda_2$, $\lambda_3$ according to directions.

More particularly, the diaphragm extraction unit 142 selects voxels having flatness higher than a reference value to extract the diaphragms from the 3-dimensional CT images $I_{CT}(t_i)$ ($1 \leq i \leq N$) and the 3-dimensional ultrasound images $I_{US}(t_j)$ ($1 \leq j \leq 2$). The flatness $\mu(v)$ is defined as below.

$$\mu(v)=\phi_1(v)\phi_2(v)\phi_3(v)/\phi_{3_{max}}(v) \tag{1}$$

$\phi_1(v)$, $\phi_2(v)$ and $\phi_3(v)$ of the equation (1) are expressed as below.

$$\phi_1(v)=\left(1-\frac{\lambda_1(v)}{\lambda_3(v)}\right)^2, \quad \phi_2(v)=\left(1-\frac{\lambda_2(v)}{\lambda_3(v)}\right)^2, \quad \phi_3(v)=\sum_i \lambda_i(v)^2 \tag{2}$$

The foregoing $\lambda_1(v)$, $\lambda_2(v)$ and $\lambda_3(v)$ represent the Hessian matrix eigen values according to the location of the voxel. The flatness $\mu(v)$ is normalized to have a value between 0 and 1. The diaphragm extraction unit 142 forms a flatness map using the flatness obtained from the equations (1) and (2), and selects voxels having relatively higher flatness. In this embodiment, the diaphragm extraction unit 142 selects voxels having flatness of 0.1 or more.

The diaphragm extraction unit 142 removes small clutters by performing morphological opening for the selected voxels (morphological filtering). The morphological opening means performing erosion and dilation sequentially. The diaphragm extraction unit 142 removes edge of the area in which the voxel values exist morphologically as many as the predetermined number of voxels to contract the edge (erosion), and then expands it as many as the predetermined number of the voxels (dilation). In an embodiment of the present invention, the diaphragm extraction unit 142 contracts and expands the edge by 1 voxel.

Since the diaphragm is the largest surface in the 3-dimensional CT image and the 3-dimensional ultrasound image, the largest surface among candidate surfaces obtained by the intensity-based connected component analysis (CCA) for the voxels may be selected as the diaphragm. The voxel-based CCA is one of the methods of grouping regions in which voxel values exist. For example, the diaphragm extraction unit 142 computes the number of voxels connected to each of the voxels through a connectivity test by referring to values of voxels neighboring to the corresponding voxel (e.g., 26 voxels), and selects the voxels of which the number of connected voxels are greater than the predetermined number as candidate groups. Since the diaphragm is the widest curved surface in the region of interest, the diaphragm extraction unit 142 extracts the candidate group having the largest number of connected voxels as the diaphragm. Thereafter, the diaphragm extraction unit 142 can smoothen the surface of the diaphragm.

In other embodiments, the diaphragm extraction unit 142 extracts the diaphragm by performing the foregoing process on the 3-dimensional ultrasound images $I_{US}$ ($t_j$) ($1 \leq j \leq 2$). In addition, the diaphragm extraction unit 142 extracts the diaphragm from the 3-dimensional CT images $I_{CT}$ ($t_i$) ($1 \leq i \leq N$) based on the input information (i.e., diaphragm area setting information). More particularly, since the 3-dimensional CT image has more distinct boundaries of liver than typical ultrasound images, the diaphragm extraction unit 142 may extract the diaphragm using methods such as a commercial program for extracting liver area or a seeded region growing segmentation method.

The blood vessel extraction unit 143 extracts blood vessels from the 3-dimensional CT images $I_{CT}$ ($t_i$) ($1 \leq i \leq N$). In addition, the blood vessel extraction unit 143 extracts blood vessels from the 3-dimensional ultrasound images $I_{US}$ ($t_j$) ($1 \leq j \leq 2$).

In one embodiment, the blood vessel extraction unit 143 can perform a blood vessel extraction from the 3-dimensional CT images $I_{CT}$ ($t_i$) ($1 \leq i \leq N$) and the 3-dimensional ultrasound images $I_{US}$ ($t_j$) ($1 \leq j \leq 2$) through masking, blood vessel segmentation and classification.

More specifically, to avoid mis-extraction of the blood vessel due to mirroring artifacts, the blood vessel extraction unit 143 sets the region of interest (ROI) masking on the 3-dimensional CT images $I_{CT}$ ($t_i$) ($1 \leq i \leq N$) and the 3-dimensional ultrasound images $I_{US}$ ($t_j$) ($1 \leq j \leq 2$) by modeling the diaphragms as a polynomial curved surface. The blood vessel extraction unit 143 may remove the portions of the modeled polynomial curved surface lower than the diaphragm using the ROI masking on the 3-dimensional CT images $I_{CT}$ ($t_i$) ($1 \leq i \leq N$) and the 3-dimensional ultrasound images $I_{US}$ ($t_j$) ($1 \leq j \leq 2$). In such a case, the blood vessel extraction unit 143 may perform modeling the diaphragm as the polynomial curved surface using the least means square (LMS). However, if all of the lower portions of the modeled polynomial curved surface are eliminated, then meaningful blood vessel information may be lost at some regions due to errors of the polynomial curved surface. To avoid losing the blood vessel information, the blood vessel extraction unit 143 applies a marginal distance of about 10 voxels from the bottom of the ROI mask and then eliminates the lower portion.

The blood vessel extraction unit 143 segments blood vessel regions and non-vessel regions. To exclude the non-vessel regions with high intensity such as the diaphragm and the vessel walls, the blood vessel extraction unit 143 estimates the low intensity bound having less intensity than a reference bound value in the ROI masked image, and removes voxels having higher intensity than the reference bound value. The blood vessel extraction unit 143 binarizes the remaining regions by applying an adaptive threshold scheme. The binarized regions become blood vessel candidates.

The blood vessel extraction unit 143 removes non-vessel-type clutters to classify real blood vessels from the blood vessel candidates. The process of blood vessel classification includes a size test for removing small clutters, a structure-based vessel test that removes non-vessel type by evaluating the goodness of fit (GOF) to a cylindrical tube (i.e., initial vessel test), gradient magnitude analysis and a final vessel test for perfectly removing the clutters. An initial threshold $C_{initial}$ is marginally set such that all blood vessels are included even if some clutters are not removed in the structure-based vessel test. In this embodiment, the initial threshold is set to 0.6. As the final vessel test, the blood vessel extraction unit 143 considers the variation of voxel values (i.e., gradient magnitude), and precisely removes all of the clutters formed by shading artifacts having low gradient magnitudes to extract the blood vessel. In this embodiment, a threshold of the final vessel test is set to 0.4.

In other embodiments, the blood vessel extraction unit 143 extracts blood vessels by performing the process described above on the 3-dimensional ultrasound images $I_{US}$ ($t_j$) (1≤j≤2). Further, the blood vessel extraction unit 143 extracts blood vessel from the 3-dimensional CT images $I_{CT}$ ($t_i$) (1≤i≤N) based on the input information (i.e., blood vessel area setting information) provided from the user input unit. More specifically, using the characteristic that blood vessels have brighter pixel value than the tissues in the liver area in the 3-dimensional CT angiography image, the blood vessel extraction unit 143 set a value of 255 only to the pixels having the value between a first threshold (T1) and a second threshold (T2), and set a value of 0 to the rest of the pixels. This process is referred to as intensity thresholding using two thresholds. As a result of this process, areas that have bright pixel values such as ribs and kidneys other than the object of interest (i.e., blood vessel) are also appeared. To remove these non-vessel regions, the blood vessel extraction unit 143 uses the connectivity of the blood vessels. Typically, the blood vessels within the liver area are composed of the portal vein and hepatic vein. Thus, the blood vessel extraction unit 143 extracts only the blood vessels by entering two specific location corresponding to each of the blood vessels as seed points and performing the seeded region growing method using the seed points as starting points.

The diaphragm refining unit 144 refines the diaphragms on the 3-dimensional ultrasound images $I_{US}$ ($t_j$) (1≤j≤2) by using the blood vessels extracted from the blood vessel extraction unit 143. More specifically, the diaphragm refining unit 144 removes the clutters by performing refinement of the diaphragm using the blood vessels extracted from the blood vessel extraction unit 143. The clutters are typically located near the vessel walls in the extracted diaphragm. Especially, the inferior vena cava (IVC) is connected to the diaphragm and causes clutters. Since these clutters may degrade the accuracy of the image registration if they are extracted as features and used in the image registration, the diaphragm refining unit 144 enhances the diaphragm by removing the clutters. The diaphragm refining unit 144 extracts the blood vessel regions from the 3-dimensional ultrasound images $I_{US}$ ($t_j$) (1≤j≤2), dilates the extracted blood vessel regions, and removes the blood vessels through which the blood is flowing to thereby estimate the vessel walls. The diaphragm refining unit 144 extracts the diaphragm by applying CCA and the size text once more.

The registration unit 145 sets sample points on anatomical features (i.e., blood vessel region and diaphragm region) for the 3-dimensional CT images $I_{CT}$ ($t_i$) (1≤i≤N) and the 3-dimensional ultrasound images $I_{US}$ ($t_j$) (1≤j≤2). The registration unit 145 then performs image registration between the 3-dimensional CT images $I_{CT}$ ($t_i$) (1≤i≤N) and the 3-dimensional ultrasound images $I_{US}$ ($t_j$) (1≤j≤2) using the set sample points to obtain the transform function $T_{probe}$ between the 3-dimensional ultrasound image and the 3-dimensional CT image. Here, the transform function $T_{probe}$ may be represented by a matrix. In this embodiment, the transform function $T_{probe}$ can be obtained by the equation (3).

$$T_{probe} = \arg\min_{X}\left[\frac{1}{N}\sum_{j=1}^{2}\min_{i}\left\{Dist(I_{US}(t_j), I_{CT}(t_i), X)\right\}\right]$$

$$(3)$$

wherein the Dist function is defined as the distance between the corresponding feature points of the 3-dimensional ultrasound image and the 3-dimensional CT image.

That is, the registration unit 145 defines the dist value with a least error between the 3-dimensional ultrasound image at maximum inspiration ($I_{US}$ ($t_1$)) and the 3-dimensional CT image ($I_{CT}$ ($t_i$)) as a first error, and defines the dist value with a least error between the 3-dimensional ultrasound image at maximum expiration ($I_{US}$ ($t_2$)) and the 3-dimensional CT image ($I_{CT}$ ($t_i$)) as a second error. Then, the registration unit 145 obtains the transform function $T_{probe}$ by calculating X that makes the sum of the first error and the second error least.

The transform unit 146 generates the transform function T for transforming the 3-dimensional CT images $I_{CT}$ ($t_i$) (1≤i≤N) based on the input information provided from the user input unit 130 and the transform function $T_{probe}$ provided from the registration unit 145. Then, the transform unit 146 acquires the 2-dimensional CT images $I_{2CT}$ ($t_i$) (1≤i≤N) by applying the generated transform function T to the 3-dimensional CT images $I_{CT}$ $(t_i)$ ($1 \leq i \leq N$).

The similarity detection unit 147 detects the similarities between the 2-dimensional ultrasound image and the 2-dimensional CT images $I_{2CT}$ $(t_i)$ ($1 \leq i \leq N$). In this embodiment, the similarities can be detected using cross correlation, mutual information, sum of squared intensity difference (SSID) and the like.

The CT image selection unit 148 selects a 2-dimensional CT image $I_{2CT}$ $(t_i)$ that has the largest similarity by comparing the similarities detected at the similarity detection unit 147.

Referring back to Figure 1, the display unit 150 displays the 2-dimensional ultrasound image provided from the ultrasound image forming unit 120 and the 2-dimensional CT image provided from the processor 140. In one embodiment, the 2-dimensional ultrasound image and the 2-dimensional CT image can be displayed in an overlapping manner. In other embodiments, the 2-dimensional ultrasound image and the 2-dimensional CT image can be displayed top and bottom or left and right on the same screen.

In the following, the process of providing the 2-dimensional CT image corresponding to the 2-dimensional ultrasound image by performing the image registration between the 3-dimensional CT image and the 3-dimensional ultrasound image with reference to the accompanying drawings. It is explained to automatically extract the diaphragm and the blood vessel from the 3-dimensional CT image for the brevity of the description. However, the present invention is not limited thereto.

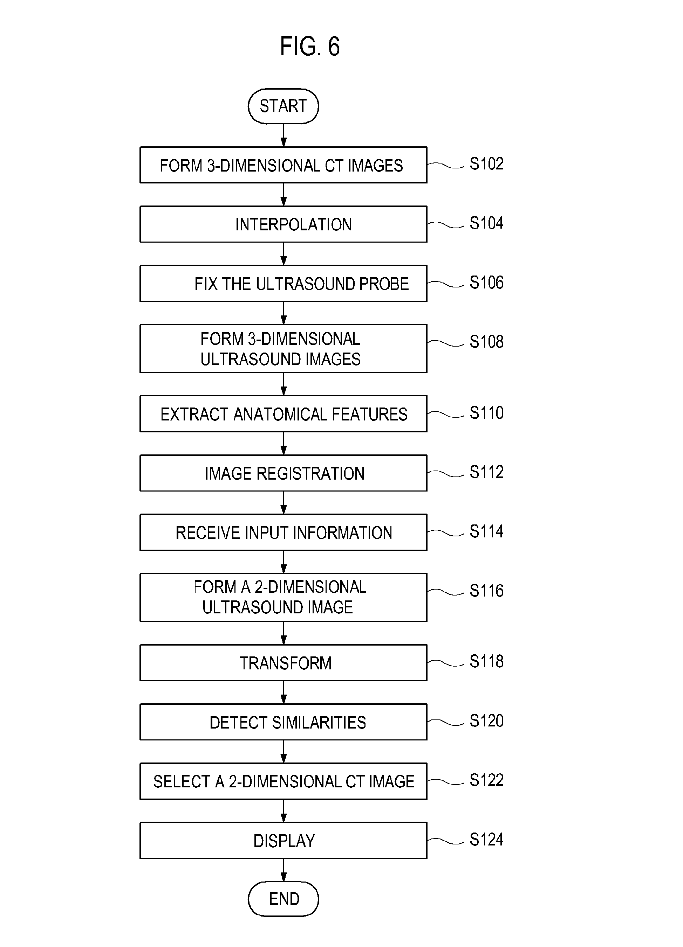

Referring to Figure 6, the CT image forming unit 110 forms 3-dimensional CT images $I_{CT}$ $(t_i)$ ($1 \leq i \leq K$) at a predetermined interval during a respiratory cycle from inspiration to expiration (S102).

The interpolation unit 141 of the processor 140 performs interpolation between the 3-dimensional CT images $I_{CT}$ $(t_i)$ ($1 \leq i \leq K$) provided from the CT image forming unit 110 to acquire 3-dimensional CT images $I_{CT}$ $(t_i)$ ($1 \leq i \leq N$) (S104).

Once the ultrasound probe 122 is fixed at the ultrasound probe holder 126 (S106), the ultrasound image forming unit 120 forms the 3-dimensional ultrasound image of the object of interest inside the target object at maximum inspiration ($I_{US}$ $(t_1)$) and the 3-dimensional ultrasound image of the object of interest inside the target object at maximum expiration ($I_{US}$ $(t_2)$) (S108).

The processor 140 extracts anatomical features (e.g., blood vessel and diaphragm) from the 3-dimensional CT images $I_{CT}$ $(t_i)$ ($1 \leq i \leq N$) and the 3-dimensional ultrasound images $I_{US}$ $(t_j)$ ($1 \leq j \leq 2$) (S110).

The registration unit 145 of the processor 140 sets sample points on anatomical features (i.e., blood vessel region and diaphragm region) for the 3-dimensional CT images $I_{CT}$ $(t_i)$ ($1 \leq i \leq N$) and the 3-dimensional ultrasound images $I_{US}$ $(t_j)$ ($1 \leq j \leq 2$). The registration unit 145 then performs image registration between the 3-dimensional CT images $I_{CT}$ $(t_i)$ ($1 \leq i \leq N$) and the 3-dimensional ultrasound images $I_{US}$ $(t_j)$ ($1 \leq j \leq 2$) using the set sample points to obtain the transform function $T_{probe}$ between the 3-dimensional ultrasound image and the 3-dimensional CT image (S 112).

Upon receiving the input information (i.e., reference plane setting information) through the user input unit 130 (S 114), the ultrasound image forming unit 120 forms the 2-dimensional ultrasound image of the section corresponding to the input information (S116).

The transform unit 146 of the processor 140 generates transform function T for transforming the 3-dimensional CT images $I_{CT}$ $(t_i)$ ($1 \leq i \leq N$) based on the input information (i.e., reference plane setting information) provided from the user input unit 130 and the transform function $T_{probe}$ provided from the registration unit 145. Then, the transform unit 146 acquires the 2-dimensional CT images $I_{2CT}$ $(t_i)$ ($1 \leq i \leq N$) by applying the generated transform function T to the 3-dimensional CT images $I_{CT}$ $(t_i)$ ($1 \leq i \leq N$) (S118).

More specifically, the transform unit 146 obtains a transform function $T_{plane}$ representing the location of the 2-dimensional ultrasound image on the 3-dimensional ultrasound images $I_{US}$ $(t_j)$ ($1 \leq j \leq N$) (i.e., location of ultrasound probe 122 for 2-dimensional ultrasound image) based on the input information provided from the user input unit 130. Here, the transform function $T_{plane}$ can be represented as a matrix. The transform unit 146 generates a transform function T for transforming the 3-dimensional CT images $I_{CT}$ $(t_i)$ ($1 \leq i \leq N$) using the transform function $T_{probe}$ and the transform function $T_{plane}$. In this embodiment, the transform unit 146 can generate the transform function T by multiplying the transform function $T_{probe}$ by the transform function $T_{plane}$. The transform unit 146 acquires the 2-dimensional CT images $I_{2CT}$ $(t_i)$ ($1 \leq i \leq N$) by applying the transform function T to each of the 3-dimensional CT images $I_{CT}$ $(t_i)$ ($1 \leq i \leq N$).

The similarity detection unit 147 of the processor 140 detects the similarities between the 2-dimensional ultrasound image provided from the ultrasound image forming unit 120 and the 2-dimensional CT images $I_{2CT}$ $(t_i)$ ($1 \leq i \leq N$) provided from the transform unit 146 (S120).

The CT image selection unit 148 selects a 2-dimensional CT image $I_{2CT}$ $(t_i)$ that has the largest similarity by comparing the similarities detected at the similarity detection unit 147 (S122). The display unit 150 displays the 2-dimensional ultrasound image provided from the ultrasound image forming unit 120 and the 2-dimensional CT image provided from the CT image selection unit 148 (S124).

While the present invention is described via some preferred embodiments, it will be appreciated by those skilled persons in the art that many modifications and changes can be made without departing the spirit and scope of the appended claims.

**Claims**

1. An image providing system, comprising:

a CT image forming unit configured to form a plurality of 3-dimensional CT images for an object of interest inside a target object;

an ultrasound image forming unit configured to form at least one 3-dimensional ultrasound image for the object of interest;

a processor configured to perform an image registration between the plurality of 3-dimensional CT images and the at least one 3-dimensional ultrasound image to obtain a first transform function; and

a user input unit configured to receive input information from a user,

wherein the ultrasound image forming unit is further configured to form a 2-dimensional ultrasound image from the at least one 3-dimensional ultrasound image based on the input information, and wherein the processor is further configured to obtain a plurality of 2-dimensional CT images from the plurality of 3-dimensional CT images based on the input information and the first transform function and to detect similarities between the 2-dimensional ultrasound image and the plurality of 2-dimensional CT images to select one of the 2-dimensional CT images corresponding to the 2-dimensional ultrasound image.

2. The system of claim 1, wherein the processor comprises:

a diaphragm extraction unit configured to extract diaphragms from the plurality of 3-dimensional CT images and the at least one 3-dimensional ultrasound image;

a blood vessel extraction unit configured to extract blood vessels from the plurality of 3-dimensional CT images and the at least one 3-dimensional ultrasound image;

a diaphragm refining unit configured to remove clutters from the diaphragms based on the blood vessels to refine the diaphragm for the at least one 3-dimensional ultrasound image;

a registration unit configured to set sample points on the blood vessels and the diaphragms for the plurality of 3-dimensional CT images and the at least one 3-dimensional ultrasound image, the registration unit being further configured to perform an image registration between the 3-dimensional CT images and the at least one 3-dimensional ultrasound image based on the sample points to obtain the first transform function;

a transform unit configured to obtain the 2-dimensional CT images from the 3-dimensional CT images based on the input information and the first transform function;

a similarity detection unit configured to detect the similarities between the 2-dimensional ultrasound image and the plurality of 2-dimensional CT images;

and

a CT image selection unit configured to select a 2-dimensional CT image having the largest similarity among the similarities.

3. The system of claim 2, wherein the diaphragm extraction unit is configured to:

calculate a degree of flatness of each of voxels of the plurality of 3-dimensional CT images and the at least one 3-dimensional ultrasound image to obtain a flatness map including degrees of flatness of the voxels;

select voxels having a higher degree of flatness than a reference value based on the flatness map to provide a 3-dimensional area comprising the selected voxels;

remove a predetermined number of morphological edge voxels from the selected voxels to contract the 3-dimensional area and expand the contracted 3-dimensional area by the predetermined number of morphological edge with voxels having a predetermined intensity to thereby remove the clutters;

obtain a plurality of candidate areas from the 3-dimensional area based on a intensity-based connected component analysis (CCA); and

select a largest area from the plurality of candidate areas to extract the diaphragm.

4. The system of claim 2, wherein the blood vessel extraction unit is configured to:

extract the blood vessels from the plurality of 3-dimensional CT images and the at least one 3-dimensional ultrasound image;

model the diaphragm as a polynomial curved surface to set region of interest (ROI) masking on the plurality of 3-dimensional CT image and the at least one 3-dimensional ultrasound image;

remove voxels having higher intensity than a reference bound value from the plurality of 3-dimensional CT images and the at least one 3-dimensional ultrasound image to select vessel candidates; and
remove non-vessel type clutters from the selected vessel candidates to classify real blood vessels.

5. The system of claim 2, wherein the input information comprises diaphragm region setting information which sets diaphragm regions on the plurality of 3-dimensional CT images and blood vessel region setting information which sets blood vessel regions on the plurality of 3-dimensional CT images.

6. The system of claim 5, wherein the blood vessel extraction unit is configured to:

extract the blood vessels from the plurality of 3-dimensional CT images based on the blood vessel setting information;
extract the blood vessels from the at least one 3-dimensional ultrasound image;
model the diaphragm as a polynomial curved surface to set region of interest (ROI) masking on the at least one 3-dimensional ultrasound image;
remove voxels having higher intensity than a reference bound value from the at least one 3-dimensional ultrasound image to select vessel candidates; and
remove non-vessel type clutters from the selected vessel candidates to classify real blood vessels, and
wherein the blood vessel extraction unit is configured to perform a structure-based vessel test, a gradient magnitude analysis, and a final vessel test for removing the non-vessel type clutters.

7. The system of claim 2, wherein the transform unit is configured to:generate a second transform function representative of a location of the 2-dimensional ultrasound image on the at least one 3-dimensional ultrasound image based on the input information;
generate a third transform function to transform the plurality of 3-dimensional CT images based on the first transform function and the second transform function; and
apply the third transform function to the plurality of 3-dimensional CT images to obtain the plurality of 2-dimensional CT images.

8. The system of claim 2, wherein the similarity detection unit is configured to calculate the similarities using one of cross correlation, mutual information and sum of squared intensity difference (SSID).

9. A method of providing an image, comprising:

forming a plurality of 3-dimensional CT images for an object of interest inside a target object;
forming at least one 3-dimensional ultrasound image for the object of interest;
performing image registration between the plurality of 3-dimensional CT images and the at least one 3-dimensional ultrasound image to obtain a first transform function;
receiving input information from a user;
forming a 2-dimensional ultrasound image from the at least one 3-dimensional ultrasound image based on the input information;
obtaining a plurality of 2-dimensional CT images from the plurality of 3-dimensional CT images based on the input information and the first transform function; and
detecting similarities between the 2-dimensional ultrasound image and the plurality of 2-dimensional CT images to select one of the 2-dimensional CT images corresponding to the 2-dimensional ultrasound image.

10. The method of claim 9, wherein performing image registration comprises:

extracting diaphragms from the plurality of 3-dimensional CT images and the at least one 3-dimensional ultrasound image;
extracting blood vessels from the plurality of 3-dimensional CT images and the at least one 3-dimensional ultrasound image;
removing clutters from the diaphragms based on the blood vessels to refine the diaphragms for the at least one 3-dimensional ultrasound image;
setting sample points on the blood vessels and the diaphragms for the plurality of 3-dimensional CT images and the at least one 3-dimensional ultrasound image; and
performing image registration between the plurality of 3-dimensional CT images and the at least one 3-dimensional ultrasound image based on the sample points to obtain the first transform function.

**11.** The method of claim 10, wherein extracting diaphragms comprises:

calculating a degree of flatness of each of voxels of the plurality of 3-dimensional CT images and the at least one 3-dimensional ultrasound image to obtain a flatness map including degrees of flatness of the voxels;
selecting voxels having a higher degree of flatness than a reference value based on the flatness map to provide a 3-dimentional area comprising the selected voxels;
removing a predetermined number of morphological edge from the selected voxels to contract or eliminate the 3-dimensional area and expanding the contracted 3-dimensional area by the predetermined number of morphological edge with voxels having a predetermined intensity to thereby remove the clutters;
obtaining a plurality of candidate areas from the 3-dimensional area based on a intensity-based connected component analysis (CCA); and
selecting a largest area from the plurality of candidate areas to extract the diaphragm, and
wherein extracting blood vessels comprises:

extracting the blood vessels from the plurality of 3-dimensional CT images and the at least one 3-dimensional ultrasound image;
modeling the diaphragms as a polynomial curved surface to set region of interest (ROI) masking on the plurality of 3-dimensional CT image and the at least one 3-dimensional ultrasound image ;
removing voxels having higher intensity than a reference bound value from the plurality of 3-dimensional CT images and the at least one 3-dimensional ultrasound image to select vessel candidates; and
removing non-vessel type clutters from the selected vessel candidates to classify real blood vessels.

**12.** The method of claim 10, further comprising, prior to performing image registration, receiving diaphragm region setting information which sets diaphragm regions on the plurality of 3-dimensional CT images and blood vessel region setting information which sets blood vessel regions on the plurality of 3-dimensional CT images.

**13.** The method of claim 12, wherein extracting a diaphragm comprises:

extracting the diaphragms from the plurality of 3-dimensional CT images based on the diaphragm region setting information;
calculating a degree of flatness of each of voxels of the plurality of at least one 3-dimensional ultrasound image to obtain a flatness map including degrees of flatness of the voxels;
selecting voxels having a higher degree of flatness than a reference value based on the flatness map to provide a 3-dimensional area comprising the selected voxels;
removing a predetermined number of morphological edge voxels from the selected voxels to contract or eliminate the 3-dimensional area and expand the contracted 3-dimensional area by the predetermined number of morphological edge with voxels having a predetermined intensity to thereby remove the clutters;
obtaining a plurality of candidate areas from the 3-dimensional area based on a intensity-based connected component analysis (CCA); and
selecting a largest surface from the plurality of candidate areas to extract the diaphragm,
wherein extracting a blood vessel comprises:

extracting the blood vessels from the plurality of 3-dimensional CT images based on the blood vessel setting information; and
extracting the blood vessels from the at least one 3-dimensional ultrasound image,
wherein the extracting the blood vessels from the at least one 3-dimentional ultrasound image further comprises:

modeling the diaphragm as a polynomial curved surface to set region of interest (ROI) masking on the at least one 3-dimensional ultrasound image;
removing voxels having higher intensity than a reference bound value from the at least one 3-dimensional ultrasound image to select vessel candidates; and
removing non-vessel type clutters from the selected vessel candidates to classify real blood vessels.

**14.** The method of claim 10, wherein obtaining a plurality of 2-dimensional CT images comprises:

generating a second transform function representative of a location of the 2-dimensional ultrasound image on the at least one 3-dimensional ultrasound image based on the input information;

generating a third transform function to transform the plurality of 3-dimensional CT images based on the first transform function and the second transform function; and

applying the third transform function to the plurality of 3-dimensional CT images to obtain the plurality of 2-dimensional CT images.

**15.** The method of claim 10, wherein detecting similarities comprises:

calculating the similarities using one of cross correlation, mutual information and sum of squared intensity difference (SSID); and

comparing the generated similarities to select the 2-dimensional CT image having largest similarity.

# FIG. 1

<u>100</u>

120

ULTRASOUND
IMAGE FORMING
UNIT

110                                              140           150

CT IMAGE
FORMING
UNIT              PROCESSOR              DISPLAY
UNIT

USER
INPUT UNIT

130

# FIG. 2

<u>120</u>

121

TRANSMISSION
SIGNAL FORMING
UNIT

123              124              125

ULTRASOUND
PROBE              BEAM
FORMER              ULTRASOUND
DATA FORMING
UNIT              IMAGE
FORMING
UNIT

122

FIG. 3

# FIG. 4

EP 2 293 245 A1

141

140

```
┌──────────────┐
│ INTERPOLATION│
│     UNIT     │
└──────────────┘
       │
       ▼
┌──────────┐   ┌──────────────┐   ┌──────────┐   ┌──────────────┐   ┌───────────┐   ┌─────────────┐   ┌──────────────┐
│ DIAPHRAGM│   │ BLOOD VESSEL │   │ DIAPHRAGM│   │ REGISTRATION │   │ TRANSFORM │   │  SIMILARITY │   │   CT IMAGE   │
│EXTRACTION│ → │  EXTRACTION  │ → │ REFINING │ → │     UNIT     │ → │    UNIT   │ → │  DETECTION  │ → │  SELECTION   │
│   UNIT   │   │     UNIT     │   │   UNIT   │   │              │   │           │   │     UNIT    │   │     UNIT     │
└──────────┘   └──────────────┘   └──────────┘   └──────────────┘   └───────────┘   └─────────────┘   └──────────────┘
```

142          143          144          145          146          147          148

FIG. 5

$$|\lambda_3|$$

$$|\lambda_1|$$

$$|\lambda_2|$$

V

plate image

$|\lambda_1|$ : Very low

$|\lambda_2|$ : Very low

$|\lambda_1|$ : High

# FIG. 6

```
        ( START )
           │
           ▼
┌─────────────────────────────┐
│ FORM 3-DIMENSIONAL CT IMAGES │────  S102
└─────────────────────────────┘
           │
           ▼
┌─────────────────────────────┐
│        INTERPOLATION         │────  S104
└─────────────────────────────┘
           │
           ▼
┌─────────────────────────────┐
│    FIX THE ULTRASOUND PROBE  │────  S106
└─────────────────────────────┘
           │
           ▼
┌─────────────────────────────┐
│        FORM 3-DIMENSIONAL    │────  S108
│       ULTRASOUND IMAGES      │
└─────────────────────────────┘
           │
           ▼
┌─────────────────────────────┐
│   EXTRACT ANATOMICAL FEATURES│────  S110
└─────────────────────────────┘
           │
           ▼
┌─────────────────────────────┐
│      IMAGE REGISTRATION      │────  S112
└─────────────────────────────┘
           │
           ▼
┌─────────────────────────────┐
│    RECEIVE INPUT INFORMATION │────  S114
└─────────────────────────────┘
           │
           ▼
┌─────────────────────────────┐
│       FORM A 2-DIMENSIONAL   │────  S116
│       ULTRASOUND IMAGE       │
└─────────────────────────────┘
           │
           ▼
┌─────────────────────────────┐
│          TRANSFORM           │────  S118
└─────────────────────────────┘
           │
           ▼
┌─────────────────────────────┐
│      DETECT SIMILARITIES     │────  S120
└─────────────────────────────┘
           │
           ▼
┌─────────────────────────────┐
│  SELECT A 2-DIMENSIONAL CT IMAGE │────  S122
└─────────────────────────────┘
           │
           ▼
┌─────────────────────────────┐
│           DISPLAY            │────  S124
└─────────────────────────────┘
           │
           ▼
        ( END )
```

Europäisches
Patentamt

European
Patent Office

Office européen
des brevets

## EUROPEAN SEARCH REPORT

**Application Number**

EP 10 17 0226

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | LEE D ET AL: "Sensorless and real-time registration between 2D ultrasound and preoperative images of the liver" 2010 7TH IEEE INTERNATIONAL SYMPOSIUM ON BIOMEDICAL IMAGING: FROM NANO TO MACRO, ISBI 2010 - PROCEEDINGS - 2010 7TH IEEE INTERNATIONAL SYMPOSIUM ON BIOMEDICAL IMAGING: FROM NANO TO MACRO, ISBI 2010 - PROCEEDINGS 2010 IEEE COMPUTER SOCIETY USA, 14 April 2010 (2010-04-14), pages 388-391, XP002602573 DOI: DOI:10.1109/ISBI.2010.5490329 * the whole document * ----- | 1,9 | INV. G06T7/00 |
| X | US 2008/212858 A1 (BOESE JAN [DE] ET AL) 4 September 2008 (2008-09-04) | 1,9 | |
| Y | * paragraph [0023] * * paragraph [0032] - paragraph [0037] * ----- | 2-8, 10-15 | |
| A | JI S ET AL: "Coregistered volumetric true 3D ultrasonography in image-guided neurosurgery" PROGRESS IN BIOMEDICAL OPTICS AND IMAGING - PROCEEDINGS OF SPIE - MEDICAL IMAGING 2008 - VISUALIZATION, IMAGE-GUIDED PROCEDURES, AND MODELING 2008 SPIE US, vol. 6918, 17 February 2008 (2008-02-17), XP040435164 DOI: DOI:10.1117/12.770382 * page 1 - page 3 * ----- -/-- | 1,9 | **TECHNICAL FIELDS SEARCHED (IPC)** G06T |

The present search report has been drawn up for all claims

| Place of search | Date of completion of the search | Examiner |
|---|---|---|
| Munich | 4 October 2010 | Rockinger, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 10 17 0226

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EADIE L H ET AL: "A real time pointer to a pre-operative surgical planning index block of ultrasound images for image guided surgery" PROCEEDINGS OF SPIE - THE INTERNATIONAL SOCIETY FOR OPTICAL ENGINEERING - VISUALIZATION AND DATA ANALYSIS 2004 2004 SPIE US, vol. 5295, 19 January 2004 (2004-01-19), pages 14-23, XP40254280 DOI: DOI:10.1117/12.526863 * the whole document * | 1,9 | |
| Y | W. H. NAM, D. -G. KANG, D. LEE, AND J. B. RA: "Anatomical feature extraction in 3D B-mode ultrasound liver images for CT-ultrasound image registration" PROC. CARS 2008, vol. 3, Supplement 1, June 2008 (2008-06), pages 401-402, XP002602575 * the whole document * | 2-8, 10-15 | |
| | | | **TECHNICAL FIELDS SEARCHED (IPC)** |
| X | XISHI HUANG ET AL: "Dynamic 2D Ultrasound and 3D CT Image Registration of the Beating Heart" IEEE TRANSACTIONS ON MEDICAL IMAGING, IEEE SERVICE CENTER, PISCATAWAY, NJ, US, vol. 28, no. 8, 6 January 2009 (2009-01-06), pages 1179-1189, XP011249697 ISSN: 0278-0062 | 1,9 | |
| Y | * page 1179 - page 1183 * | 2-8, 10-15 | |

The present search report has been drawn up for all claims

| Place of search | Date of completion of the search | Examiner |
|---|---|---|
| Munich | 4 October 2010 | Rockinger, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                 EP 10 17 0226

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2010

| Patent document cited in search report | Publication date | Patent family member(s) | Publication date |
|---|---|---|---|
| US 2008212858 A1 | 04-09-2008 | DE 102007010806 A1 | 04-09-2008 |

For more details about this annex : see Official Journal of the European Patent Office, No. 12/82

**EP 2 293 245 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 10200970981